# EUROPEAN PATENT SPECIFICATION

(11) **EP 2 092 081 B1**
(45) Date of publication and mention of the grant of the patent: **15.02.2012**
(21) Application number: 07823247.7
(22) Date of filing: 13.11.2007
(51) Int. Cl.: C12Q 1/68

(54) **METHODS AND USES INVOLVING GENETIC ABNORMALITIES AT CHROMOSOME 12**
VERFAHREN UND VERWENDUNGEN IN VERBINDUNG MIT GENETISCHEN ANOMALIEN AUF CHROMOSOM 12
PROCEDES ET UTILISATIONS IMPLIQUANT DES ANOMALIES GENETIQUES SUR LE CHROMOSOME 12

(30) Priority: 13.11.2006 FI 20065717; 13.11.2006 US 858512 P
(43) Date of publication of application: 26.08.2009
(73) Proprietor: Helsingin yliopiston rahastot, 00100 Helsinki (FI)
(72) Inventor: KROHN, Kai, 36450 Salmentaka (FI); HASSAN, Wael, University of Sharjah, Sharjah (AE); PELTOMÄKI, Päivi, 00420 Helsinki (FI); HELLE, Markku, 50100 Mikkeli (FI); HAHTOLA, Sonja, 02600 Espoo (FI); KARENKO, Leena, 00670 Helsinki (FI); RANKI, Annamari, 00250 Helsinki (FI)
(74) Representative: Lax, Monica Ingeborg
(86) International application number: PCT/FI2007/050611
(87) International publication number: WO 2008/059112

(56) References cited:
- WO-A2-2005/005661
- WO-A2-2005/118869
- FI-B1- 113 666
- US-B2- 7 094 534
- SCHNEIDER B G ET AL: "Regions of allelic imbalance in the distal portion of chromosome 12q in gastric cancer" MP. MOLECULAR PATHOLOGY, BMJ PUBLISHING GROUP, LONDON, GB LNKD- DOI:10.1136/MP.56.3.141, vol. 56, no. 3, 1 June 2003 (2003-06-01), pages 141-149, XP008109733 ISSN: 1366-8714
- SCHNEIDER B.G. ET AL.: 'Regions of allelic imbalance in the distal portion of chromosome 12q in gastric cancer' MOL. PATHOL. vol. 56, June 2003, pages 141 - 149, XP008109733
- STORLAZZI C.T. ET AL.: 'Ring chromosome and low-grade gene amplification in an atypical lipomatous tumor with minimal nuclear atypia' INT. J. ONCOL. vol. 23, July 2003, pages 67 - 71, XP008110298
- SCHMIDT H. ET AL.: 'Gains of 12q are the most frequent genomic imbalances in adult fibrosarcoma and are correlated with a poor outcome' GENES CHROMOSOMES CANCER vol. 34, May 2002, pages 69 - 77, XP008109735

## Description

### Field of the invention

The present invention relates to the fields of genetics and oncology and provides methods for predicting and identifying tumors of epithelial origin. Specifically, the present invention relates to a novel method of predicting tumor initiation, tumor progression and/or carcinomas, the method comprising detecting genetic abnormality associated with tumors of epithelial origin. The present invention further relates to a novel method of identifying an individual with potential for developing carcinoma, the method comprising detection of genetic abnormalities. The present invention also relates to a method of predicting the progression of carcinomas and the transformation thereof to an aggressive variant, the method comprising detection of genetic abnormalities, which indicate the probability to develop carcinoma. The present invention also relates to a use of specific chromosomal region, a gene or a fragment thereof, and/or genetic markers for predicting tumor initiation, tumor progression and/or carcinoma. The present invention also relates to a use of specific chromosomal region or a gene or a fragment thereof in therapy, for the development of therapy and for the preparation of a medicament for treating tumors of epithelial origin.

### Background of the invention

Cancer is a complex disease in which several genetic and epigenetic abnormalities have accumulated. A varying number of genetic changes are needed prior to occurrence of a somatically developed tumor. Available data indicate that the development of solid tumors is dependent on combination of deletions and amplifications of multiple chromosome segments (Mertens et al. Cancer Res 57: 2765-2780, 1997; Mitelman et al. Nature Genet, 15: 417-474, 1997). Over 90% of all human neoplasia is derived from epithelia. Thus, epithelial cells play an important role in physiological and pathophysiological conditions. Carcinomas are malignant tumors derived from epithelial cells. The most common carcinomas include the common forms of breast, prostate, lung and colorectal cancer.

Colorectal cancer is the third most common cancer worldwide with an estimated one million new cases annually (Parkin et al. CA Cancer J Clin 55: 74-108, 2005). The average lifetime risk in industrialized countries is approximately 5%, and almost half of those affected will die of their disease (Burt, Gastroenterology 119: 837-853, 2000). Colorectal cancer develops via a benign precursor lesion, polyp, and is preventable through polypectomy. It is estimated that 30% of the population have colonic polyps, and the incidence of polyps increases with age. Thus, screening colonoscopies in average asymptomatic individuals have revealed neoplastic (adenomatous) polyps in 12% of individuals of 40-49 years of age (Imperiale TF et al. NEJM 346: 1781-1785, 2002), and in 58% among 50-59 year-old individuals (Mehran A et al. Surg Endosc 17: 1974-1977, 2003). Certain inherited disorders, which account for some 5-10% of the total colorectal cancer burden, are associated with an increased number of polyps (familial adenomatous polyposis, FAP) or an elevated tendency to malignant progression (hereditary nonpolyposis colorectal cancer, HNPCC) (Lynch and de la Chapelle, N Engl J Med 348: 919-932, 2003).

Survival is closely related to the stage at diagnosis, even in patients who have already developed malignant disease: over 90% of patients with local cancer are alive after 5 years as opposed to less than 10% of those with metastatic disease (Burt, Gastroenterology 119: 837-853, 2000). Colorectal carcinomas are notoriously resistant to both chemotherapy and radiotherapy and most patients for whom surgery alone is not curative are doomed to die of their disease (Globcan, International Agency for Research on Cancer. Available at http:/www-dep.iarc.fr/, 2002). It is therefore vital to be able to identify individuals with an increased risk as early as possible to enable efficient cancer prevention or curative treatment.

Colorectal cancer development via benign precursors along with the accumulation of genetic changes is one of the best-known examples of multistep carcinogenesis (Chung DC, Gastroenterology 119: 854-865, 2000, and below). This multistep evolutionary nature of colorectal cancer provides excellent opportunities for early cancer detection and prevention. Colorectal cancers arise as a result of stepwise accumulation of mutations at the nucleotide level and/or at the gross chromosomal level. The overwhelming majority of colorectal cancers display one of the two major genomic instability phenotypes, microsatellite instability (MSI) or chromosomal instability (CIN) (Abdel-Rahman et al. Proc. Natl. Acad. Sci. USA 98: 2538-2543, 2001). The current literature includes a multitude of biomarkers of potential use in colorectal cancer risk assessment or early detection of this cancer; however, clinical validation is mostly lacking (Umar and Srivastava, Dis Markers 20: 87-96, 2004).

Currently, histology serves as a main predictor, with multiplicity of adenomas, high-grade dysplasia, villous features, and large size (over 1 cm), for increased cancer risk (Winawer et al. Gastroenterology 130: 1872-1885, 2006). Therefore, predictors of advanced pathology would be useful, for both adenomas and cancer, to be able to assign an appropriate risk category for each patient. Biomarkers that could serve as predictors of a tendency to cancer progress would be highly welcome.

Lung cancer is a leading cause of cancer-related deaths worldwide, with approximately 1.2 million deaths annually (Ferlay *et al.* 2001, GLOBOCAN2000: Cancer Incidence, Mortality and Prevalence Worldwide, Version 1.0 IARC CancerBase No. 5. Lyon, IARCPress). Up to 95 percent of lung cancers are smoking related and thus, DNA adducts have a key role in carcinogenesis.

Patients suffering of lung cancer often have poor prognosis, five-year-survival rates ranging from approximately 50% to 10% (Hasleton PS, Respiratory system 1.0 in Cancer Handbook. http:www.cancerhandbook.net, London: Nature Publishing Group, 2001). However, when lung cancer is detected in an early-stage and surgery is possible, the five-year survival rates can reach 85%. Thus, predictors of tumor initiation and/or progression would be valuable, in order to enable effective cancer prevention or therapy.

Malignancies of the lung can be divided based on the histological characteristics into small cell (SCLC) and non-small cell lung cancers (NSCLC), the latter consisting mainly of epidermoid carcinoma and adenocarcinoma. Recent studies have shown the genetic background to be different among these cancer types (Kaminski et al. Chest 125 (5 Suppl): 111 S-5S, 2004, Fong et al. Thorax 58: 892-900, 2003). However, it is assumed that over 20 genetic or epigenetic abnormalities are needed before clinically evident lung cancer. Typically, in lung carcinomas, multiple chromosome aberrations can be observed indicating genomic instability. Novel tumor markers would explain the pathogenesis of cancer and therefore, improve the effect of therapies and survival in lung cancers.

Diagnostics longs for single markers or a panel of markers for general screening of cancers. For example, prostate specific antigen (PSA) is secreted by the cells of the prostate gland and elevated levels of PSA are used as a marker for prostate tumors.

Several other markers for epithelial tumors are available but their use is hampered from their nonspecificity: These markers are often elevated also in other conditions than malignancy, such as in inflammatory lesions. Such markers, that have been used in the clinics but that do not meet the requirement of specificity and/or sensitivity are, for instance the following: tumor-derived colon-specific antigen (tCSA), carcinoembryonic antigen (CEA), alpha-fetoprotein (AFP), pregnancy-specific beta-glycoprotein 1 (SP1), human placental lactogen (HPL), human beta chorionic gonadotrophin (beta-HCG), transferrin (TF) and ferritin (FE).

It is of highest importance to develop new methods, which enable early identification of patients with an increased risk to develop aggressive carcinoma to enable efficient cancer prevention. There is also a need for a clinically useful method that could serve as a predictor of a carcinoma progressing tendency. Also, additional means for the development of new guidelines for the initiation and follow-up of cancer therapy are greatly needed.

Chromosome 12q21 aberrations, specifically neuron navigator 3 *(NAV3)* gene aberrations, have been identified in neuroblastomas and cutaneous T-cell lymphoma (CTCL). Four of 10 primary neuroblastomas studied by Coy et al. showed reduced or absent expression of *NAV3,* and three of them had homozygous deletions of both alleles (Coy JF et al. Gene 290: 73-94, 2002). In CTCL, a deletion or a translocation of *NAV3* gene was associated with a point mutation in the remaining allele only in one of 7 patients studied (Karenko L et al. Cancer Res 65: 8101-8110, 2005 and EP1476567 A1). In pancreatic carcinomas, chromosome 12q21 aberrations were identified by microsatellite analysis. Loss of heterozygosity (LOH) was detected with markers D12S1684 and D12S1708, which border a chromosomal region comprising *NAV3* gene (Kimura M et al. Cancer Res 58: 2456-60, 1998). However, the chromosomal region described in the article by Kimura *et al.* is large and neither the specific region nor the NAV3 gene was observed to be linked to pancreatic carcinoma. On the contrary, this application describes chromosomal abnormalities that are specific for tumors of epithelial origin, in the specific chromosomal region.

Novel biomarkers for providing more effective and early diagnosis of potentially aggressive tumors as well as identifying tumors susceptible to targeted therapies are warranted. The present invention provides one solution for predicting or identifying tumor and carcinoma progression. The present invention also discloses a tool for evaluating clinical aggressiveness of epithelial tumors and patient survival. Furthermore, the invention provides a new therapeutic target for carcinoma prevention or therapy.

### Brief description of the invention

The object of the invention is thus to provide novel methods and means for diagnosing, staging and monitoring of patients having cancer, such methods and means allowing an early diagnosis of the disease.

Another object of the invention is to provide novel methods and means for predicting tumor initiation, tumor progression and/or carcinoma.

Another object of the invention is to provide novel methods and means for identification of individuals with an increased risk to develop carcinomas, such methods and means being specific and reliable and allowing identification as early as possible.

Yet another object of the invention is to provide novel methods and means for the prediction of the progression of carcinomas and the transformation to an aggressive form, such methods and means allowing a timely therapeutic intervention, which may be life-saving.

Still another object of the invention is to provide novel methods and means for the development of new guidelines for the initiation and follow-up of therapeutic interventions as well as for the development of new treatment modalities for cancers, such methods and means prolonging the remission stage of the disease and introducing new possibilities for combating the disease and for the recovery of the patient.

Still another object of the invention is to provide novel biomarkers useful in early detection of the cancer as well as cancer risk assessment.

The present invention relates to a novel method for prediction of tumor initiation, tumor progression and/or carcinomas, characterized by detecting the presence or the absence of genetic abnormalities at 12q21.2 in a neuron navigator 3 (NAV3) gene or a fragment thereof, the presence of said genetic abnormalities being associated with tumors of epithelial origin, in a biological sample. In other words, the genetic abnormalities indicate the presence of epithelial tumors or an initiation or progression of tumors of epithelial origin and/or carcinoma.

The present invention further relates to a novel method for identifying an individual with potential for developing carcinoma of epithelial origin, the method comprising detection of genetic abnormalities at 12q21.2 in a neuron navigator 3 (NAV3) gene or a fragment therof, said genetic abnormalities being associated with tumors of epithelial origin. That is, the genetic abnormalities indicate tumors of epithelial origin with potential for developing carcinoma.

The present invention further relates to a novel method of predicting the progression of carcinomas of epithelial origin and/or the transformation thereof to an aggressive variant, characterized by detecting genetic abnormalities at 12q21.2 in a neuron navigator 3 (NAV3) gene or a fragment therof, wherein abnormalities indicate the probability to develop carcinoma.

The present invention also relates to the use of the *NAV3* gene or a fragment thereof for predicting tumor initiation, tumor progression and/or carcinoma, genetic abnormalities in the *NAV3* gene indicating tumors of epithelial origin.

The present invention also relates to the use of genetic markers at 12q21.2, for predicting tumor initiation, tumor progression and/or carcinoma, characterized by detecting the presence or absence of genetic abnormalities, said genetic abnormalities at 12q21.2 in *NAV3* gene or in a fragment thereof indicating tumors of epithelial origin. Said genetic abnormalities are associated with tumors of epithelial origin and/or carcinoma.

The present invention also describes the use of a specific chromosomal region 12q21.2, specifically the *NAV3* gene, a fragment thereof or a gene product thereof in therapy, for the development of therapy or for the preparation of a medicament for treating tumors of epithelial origin.

### Brief description of the drawings

In the following the invention will be described in greater detail by means of preferred embodiments with reference to the attached drawings, in which
Figure 1 shows a LOH observed at the chromosome 12 microsatellite D12S1708 in both adenoma (middle) and carcinoma (bottom) as compared to their matching normal tissue (top).
Figure 2 shows a MSI observed at the chromosome 12 microsatellite D12S1708 in the carcinoma (bottom) compared to its matching normal tissue (top). The adenoma (middle) is MSS.
Figure 3 shows single nucleotide primer extension, (SnuPE) showing LOH in a carcinoma (bottom) as compared to its matching normal (top).
Figure 4 shows a karyotype of the true malignant lung carcinoma cells of a patient with CTCL and SCLC.
Figure 5a shows the result of *NAV3*-specific FISH with breast cancer metastases. Black bars indicate the amount of polyploidy in studied cells and grey bars indicate the amount of *NAV3* deleted cells. Results are shown as percentage of total cell count.
Figure 5b shows typical cells of breast cancer metastases with *NAV3* deletion. Green signals indicate centromeres and red signals *NAV3* copies.
Figure 6 shows comparison of *NAV3* FISH results from normal colon and colon cancer samples. *NAV3* FISH analysis included both normal colon and CRC samples from the same patient (n=36). Mean values (%) of normal colon (grey bars) and colon cancer (black bars) are shown.

### Detailed description of the invention

It has been found that abnormalities at 12q21.1-q21.31, specifically 12q21.2, are associated with tumors of epithelial origin.

Chromosome 12q21.1-q21.31 aberrations, specifically 12q21.2 aberrations, more specifically *NAV3* gene aberrations, have been found to have role in the development of epithelial originated tumors.

The present invention is based on a method for detecting genetic abnormalities at 12q21.1-q21.31, specifically 12q21.2, associated with tumors of epithelial origin, excluding carcinoma of pancreas.

Specifically, genetic abnormalities at chromosomal position 12q21.1-q21.31, specifically 12q21.2, affect *NAV3* gene or a fragment thereof.

Specifically, genetic abnormalities are detected in *NAV3* gene or a fragment thereof.

In one preferred embodiment of the method of the invention the tumor of epithelial origin is an adenoma and/or a carcinoma.

In another preferred embodiment of the method of the invention the location of the tumor of epithelial origin is colon, rectum, lung, urinary bladder, breast, squamous or basal cells. In other words, the epithelial tumor is a colon tumor, rectum tumor, lung tumor, urinary bladder tumor, breast tumor, squamous cell tumor or a basal cell tumor. In the large intestine, the colorectal tumors can be either adenocarcinomas or premalignant adenomas or polyps, in the urinary bladder the tumor can be transitional epithelial polyps with poor differentiation or overt transitional carcinomas, the breast tumors can be either ductal carcinomas or acinar carcinomas and in the skin, the tumors can be either basaliomas or epidermoid carcinomas (also called squamous cell carcinoma or spinocellular carcinoma). In the lung, the tumor can be either epidermoid carcinoma or adenocarcinomas.

In a further preferred embodiment of the method of the invention the genetic abnormalities are determined by the loss of heterozygosity (LOH) of *NAV3* gene or a fragment thereof, wherein LOH of *NAV3* is indicative of tumor progression.

In a further preferred embodiment of the method of the invention the genetic abnormalities of *NAV3* gene are determined in haploid, diploid and/or polyploid cells.

In a further preferred embodiment of the method of the invention the tumor cells are microsatellite stable (MSS) or microsatellite instable (MSI).

In a further preferred embodiment of the method of the invention the tumor of epithelial origin is other than carcinoma of pancreas.

The present invention is also based to the use of chromosomal region 12q21.1-q21.31, specifically 12q21.2, *NAV3* gene or a fragment thereof, and/or markers at 12q21.1-q21.31, specifically 12q21.2, associated with tumors of epithelial origin.

In one preferred embodiment the markers at 12q21.1-q21.31 include D12S1684, D12S326, D12S1708 and/or rs1852464.

In one preferred embodiment the markers at 12q21.2 include D12S326 and/or rs1852464.

As used herein the expression "genetic abnormality" refers to the presence of a translocation, deletion, amplification, inversion or another defect at 12q21.1-q21.31, specifically at 12q21.2.

As used herein the expression "deletion" refers to the absence of a nucleotide or nucleotides and/or an exon or exons in the gene sequence which absence adversely affects the function of the gene. The expression also refers to the absence of the gene fragment, gene or the chromosomal fragment containing the gene.

As used herein the expression "another defect" refers to any genetic alteration, such as a substitution, an addition, polymorphism, insertion, inversion etc., which is associated with tumors of epithelial origin.

As used herein the expression "loss of heterozygosity (LOH)" refers to the loss of a single parent's contribution to part of the cell's genome. LOH can be considered as an event to unmask a mutant allele of a gene which may play a role in suppressing tumor formation. Thus, LOH is an important marker for tumor initiation or progression.

As used herein the expression "BLOH" refers to borderline LOH, meaning that one of the alleles in the tumor sample has 25%-39% signal reduction compared to its matching normal.

As used herein the expression "translocation" refers to transfer of chromosomal regions between non-homologous chromosomes.

As used herein the expression "amplification" refers to gain of genetic material such as a gene fragment, a gene or the chromosomal fragment containing the gene.

As used herein the expression "tumor" refers to an abnormal mass of tissue due to abnormal excess of cells divisions or lack of normal cell death. Tumors may be benign or malignant, in other words not cancerous or cancerous. Tumors include such as adenomas, carcinomas or polyps.

As used herein the expression "adenoma" refers to a noncancerous tumor.

As used herein the expression "carcinoma" refers to a cancer of epithelial origin.

As used herein the expression "epithelial" refers to the cells that line the internal and external surfaces of the body.

As used herein the expression "tumors of epithelial origin" refers to tumors, which arise from epithelial cells. The tumors of epithelial origin include such as breast, colorectal, lung, urinary bladder, breast, squamous cell, basal cell, prostate, gastric, esophagus and mouth/tongue tumors.

Epithelial tumors arise from epithelium, the specified set of cells that cover organs and surfaces of the body. Epithelium can be simple, such as the one cell layer epithelium covering part of respiratory tract, mammary gland ducts and ductuli or intestine, or can be stratifies, composed of several layers of cells, such as is found in the upper layer of skin or in the urinary bladder. The epithelium of the skin is keratinizing, meaning that while the basal cells of the epidermis, covering skin are round and proliferate, the uppermost cells are flattened, non-dividing and their cytoplasm is filled with keratin fibres. The urinary epithelium, on the other hand, is not keratinizing but even here, the basally located cells are round while the cells located closer to the surface are flattened and thus, this type of epithelium is called transitional.

As used herein the expression "indicating tumors of epithelial origin" refers to that the presence or high probability or possibility of epithelial tumors is shown or described or proved or evidenced.

As used herein the expression "aggressive variant" refers to a cancer, which grows fast and possibly metastasizes.

As used herein the expressions "fragment" or "functional fragment" refer to a part of *NAV3* gene, which is detectable in the methods of the invention such as LOH-analysis or FISH-methods.

As used herein the expression "gene product" refers to a mRNA, protein or to any product achieved directly or indirectly from the gene.

Neuron navigator 3 (*NAV3* or *POMFIL1*) gene is a member of a recently identified human gene family, which shows homology to the *unc-53,* an axonguidance gene from *Caenorhabditis elegans* (Maes et al. Genomics 80: 21-30, 2002). It also shares homologous sequences with human RAINB1 (retinoic acid inducible in neuroblastoma cells) a mammalian homologue of unc-53 (Merrill et al. PNAS 99: 3422-3427, 2002). By structure prediction NAV3 has calponin-like domains and SH3 binding sites suggestive of a role in cell signaling (Coy JF et al. Gene 290: 73-94, 2002 and Maes et al. Genomics 80: 21-30, 2002) *NAV3* consists of 39 exons and its expression, based on mRNA detection, is largely restricted to the brain tissue (Maes et al. Genomics 80: 21-30, 2002). *NAV3* was shown to produce transcripts encoding proteins of different lengths and it may be subject to tissue-specific alternative splicing. NAV3 is structrally a helicase and exonuclease, resembling Wemer and Bloom syndrome proteins with the role in maintaining stability of chromosomes (Coy JF et al. Gene 290: 73-94, 2002, Maes et al. Genomics 80: 21-30, 2002). Subcellularly, NAV3 has been reported to locate in nuclear pre complexes (Coy JF et al. Gene 290: 73-94, 2002) and might have a role in nuclear transport, kinetochore formation and cell cycle control (Fahrenkrog B and Aebi U, Nat Rev Mol Cell Biol 4: 757-66, 2003). Thus, NAV3 could be a non-classical haploinsufficient tumour suppressor (Sherr CJ, Cell 116: 235-46, 2004).

In the present invention, genetic abnormalities at 12q21.1-q21.31, specifically 12q21.2, were studied by LOH analysis for colorectal adenomas, carcinomas lung cancers and urinary bladder cancer. Fluorescence *in situ* hybridization (FISH) was also utilized for colon tumors, breast cancer, basal cell carcinoma (BCC) and squamous cell carcinoma (SCC), and comparative genomic hybridization (CGH) for lung cancers in order to scrutinize chromosomal position 12q21.1-q21.31, specifically 12q21.2.

All the microsatellite markers (D12S1684, D12S326, D12S1708) as well as the SNP marker (the intragenic *NAV3* rs1852464) showed LOH at 12q21.1-q21.31 in adenomas and carcinomas of colorectum. In urinary bladder cancer samples, at least borderline LOH was detected with said four markers. Microsatellite markers also showed LOH in lung cancers. Furthermore, FISH revealed loss of 12q21 in colon tumors and both loss and gain of 12q21 in breast cancers, basal cell carcinoma and squamous cell carcinoma. CGH revealed loss of 12q21 in one lung cancer. Thus, loss or gain of *NAV3* appears as a marker of tumors originating from epithelia.

In addition, colorectal adenomas and carcinomas arising in the same patient showed *NAV3* LOH suggesting that adenoma patients will develop carcinomas through *NAV3* LOH. Poor differentiation was observed in an adenoma with *NAV3* LOH and the size of *NAV3* LOH adenomas tended to be greater than of those without LOH.

We have now observed, that the chromosomal abnormalities, found in epithelial tumors, in fact do occur in tumors arising in all different types of epithelium. Thus, the basaliomas (also called carcinoma basocellulare or basal cell carcinomas) are formed from the cells normally located in the most basal part of epidermis. On the other hand, the squomous cells carcinoma (also called carcinoma squamocellulare or spinocellular carcinoma), are formed from the more distally located cells and this tumor often show keratinisation, a feature characteristic for the keratinizing cells. Carcinomas of breast and colorectal carcinomas are examples of malignant tumors arising from simple epithelium, having only one layer of cells normally, but even here, the original benign epithelium contains differentiated cell types, such as exocrine cells of the mammary gland, secreting either milk or mucus, or mucus secreting Goblet cells in the gut epithelium.

Epithelial tumors can be benign, premalignant or overtly malignant. We did observe the chromosomal abnormality described in more detail in this application mostly in malignant tumours, carcinomas but also in some of the premalignant conditions, such as large adenomas of colon or rectum.

Because genetic abnormalities were associated with tumors of epithelial origin, the presence of those abnormalities indicate the initiation, progression and/or presence of tumors of epithelial origin or the development, progression and/or presence of carcinomas. Therefore, it is possible to diagnose or identify the patients who have the tumor of the epithelial origin or carcinoma by detecting the presence of said aberrations in a biological sample. It is also possible to diagnose or identify the patients whose tumors are likely to progress or develop or transform to an aggressive variant by detecting the presence of said aberrations. The biological samples from the patients or suspected patients can be screened for the presence of said genetic abnormalities.

According to the method of the present invention, the presence or absence of genetic abnormalities can be detected from a biological sample by any known detection method suitable for detecting translocations, deletions, insertions etc. Such methods are easily recognized by those skilled in the art and include fluorescence *in situ* hybridisations, such as multi-colour fluorescence in situ hybridisations, multi-fluor *in situ*-hybridisation (MFISH), spectral karyotyping (SKY), Combined binary ratio labelling (COBRA), colour changing karyotyping (CCK). In comparative genomic hybridization (CGH) the genetic changes are classified as DNA gains and losses. CGH reveals a characteristic pattern that includes aberrations at chromosomal and subchromosomal levels. The conventional G-banding techniques can also be used in cases were the coarse detection of gains, losses or translocations is regarded as sufficient. Preferable methods are those suitable for use in clinical laboratories.

According to one preferred embodiment of the present invention, which takes advantage of the identification of *NAV3* gene in tumors of epithelial origin, the presence or absence of the *NAV3* gene or an equivalent or a fragment thereof can be detected from a biological sample by any known detection method suitable for detecting a gene expression (or copy number), i.e. methods based on detecting the copy number of the gene (or DNA) and/or those based on detecting the gene expression products (mRNA or protein). Such methods are easily recognized by those skilled in the art and include conventional polymerase chain reaction (PCR)-methods, RT-PCR, *in situ* hybridisations, such as FISH, mRNA *in situ* hybridisation, Northern analysis, Southern and Western analyses, immunohistochemistry, and other immunoassays, such as ELISA. Preferable methods are those suitable for use in routine clinical laboratories.

According to another preferred embodiment of the present invention, which takes advantage of the LOH analysis for detecting abnormalities of *NAV3* gene, deletion, gene conversion, mitotic recombination and chromosome loss can be detected. LOH in cancers can be identified by the presence of heterozygosity at a genetic locus in germline DNA and the absence of heterozygosity at the same locus in the tumor cells.

According to another preferred embodiment of the present invention, which takes advantage of the markers suitable for detecting abnormalities of *NAV3* gene, markers include any biological markers such as microsatellite markers, SNP-markers, any probes, primers or antibodies associated with *NAV3* gene. Numerous methods are suitable for analysing nucleic acids for the presence of specific sequence variations such as polymorphisms, SNP's, insertions or deletions. Allelic variants can be discriminated for example by enzymatic methods, electrophoretic methods, and physical methods. These methods include for example single strand conformation polymorphism (SSCP), heteroduplex analysis, fragment analysis, DNA sequencing, minisequencing, primer extension methods, microarrays, mass spectrometry and denaturing high performance liquid chromatography (DHPLC). PCRs are often used in analyzing specific sequence variations or exploited in combination with aforementioned methods.

In the method of the invention, the biological sample can be any suitable tissue sample, such as biopsy from the epithelial tissue or lymph node or a metastatic tumor lesion in any body organ or whole blood. The biological sample can be, if necessary, pretreated in a suitable manner known to those skilled in the art.

In therapy, restoration of the normal function of the *NAV3* gene can be used. This may be reached by enhancing the expression of functionally homologous genes, by introducing an intact *NA V3* gene or by using an altered form of the *NAV3* gene or antisense oligonucleotide against the *NAV3* in any technique presently available for gene therapy to prevent the progression of a proliferating disease. In particular, tumor cell growth may be slowed down or even stopped by such therapy. Such techniques include the *ex vivo* and *in situ* therapy methods, the former comprising transducing or transfecting an intact or altered *NA V3* gene (or its functional domains) in a recombinant or peptide form or as antisense oligonucleotides or in a vector to the patient, and the latter comprising inserting the altered gene or oligonucleotide into a carrier, which is then introduced into the patient. Depending on the disease to be treated, a transient cure or a permanent cure may be achieved. Alternatively, monoclonal or humanized antibodies or peptides binding to the NAV3 protein or to the fusion gene generated as a result of the translocation, can be used to suppress the function of the altered NAV3 protein and thus tumor cell growth may be slowed down or even stopped. Antibodies against NAV3 could also be used to carry other agents, such as cytotoxic substances, to the cancer cells over-expressing the *NAV3* gene. Such agents could then be used to kill specifically the cancer cells.

Understanding the genetic aberrations or the chromosomal changes, especially those associated with the tumor initiation will contribute to early diagnosis of cancer and treatment of patients. The present invention discloses for the first time the role of *NAV3* LOH in epithelial tumors. The present invention also discloses that when *NAV3* LOH is observed in colorectal adenomas it is likely that such patient will develop carcinomas through *NAV3* LOH as well.

Detection of deletions or other defects of the *NAV3* gene as described in the present invention, allows thus earlier identification of patients with an increased risk to develop aggressive cancer and enables efficient cancer prevention and development of novel diagnostic and follow-up of carcinomas, such as colorectal or lung cancer. Discovery of genetic abnormalities of *NAV3* in epithelial tumors also opens new possibilities in the advancement of therapies thereof.

The following examples are given for further illustration of the invention.

It will be obvious to a person skilled in the art that, as the technology advances, the inventive concept can be implemented in various ways. The invention and its embodiments are not limited to the examples described below but may vary within the scope of the claims.

### Example 1

### a) NAV3 loss of heterozygosity (LOH) analysis using microsatellite markers

Histology of the formalin-fixed paraffin-embedded tissue samples was verified by a histopathologist. Tumors, adenomas or normal areas were dissected out to get pure normal or at least 50% ratio of carcinoma or adenoma tissue for the DNA preparation according to a standard protocol. Paraffin embedded sections were cut at 10 µm thickness and DNA was purified from these following standard protocols (Isola et al. Am J Pathol 145: 1301-1308, 1994).

LOH analysis was performed for *NAV3* gene. Three microsatellite markers spanning the *NAV3* gene locus at 12q21.1-q21.31 and surrounding the gene from both directions (physical distances between loci in mega-bases according to http://www.ensembl.org are given in parentheses) were chosen: pter D12S1684 -(0.8 Mb)- D12S326 -(0.2 Mb)- NAV3 -(3.8 Mb)- D12S1708 qter. The DNA samples were amplified by polymerase chain reaction (PCR) using the following primers: D12S1684F 5'cctgcatgcctcagttatga3', D12S1684R 5'aacaagccataccagtcagg3', D12S326F 5'accaggctcccctaaaagtg3', D12S326R 5'agaatgaccagacccacagg3', D12S1708F 5'gggaacttatgtcaaggctagga3', D12S1708R 5'gatctagtgctcaagaggttttcaa3'. PCR reactions were performed in 25-µl reaction volume containing 75-150 ng of template DNA, GeneAmp 10x PCR buffer (Applied Biosystems), 0.2 mM of dNTP Mix (GE Healthcare Biosciences Ab), 0.8 umol of each primer, and 1,5 U of AmpliTaq polymerase (AB). The following PCR cycles were used for amplification: 94°C for 3 minutes, 35 cycles of 94°C for 30 seconds denaturation, annealing temperature of 60°C for 30 seconds, and 72°C for 45 seconds extension. Final extension was 72°C for 5 minutes. The forward primers were fluorescently labeled with FAM and PCR fragments were run on the ABI3730 sequencer/genotyper and results analysed using GeneMapper v3 software (Applied Biosystems).

### b) NAV3 loss of heterozygosity (LOH) analysis using single nucleotide primer extension, SnuPE

The DNA was prepared as above.

A non-radioactive method was used to quantify the relative expression of the two *NAV3* alleles in patients heterozygous for the coding A/G polymorphism (rs1852464) within exon 19 of the *NAV3* gene. The heterozygosity for rs1852464 is up to 0.493 in the Caucasians/Europeans making it a highly useful marker. The extension reaction of SNuPE is based on the incorporation of a single ddNTP that is selected to allow differential extension of a labeled primer annealed close to the polymorphic site.

Matching tumor and normal genomic DNA samples from the same individuals were first PCR amplified using primers rs1852464F 5' CCTGCTATTTTCATCTTTCAAGC 3' and rs1852464R 5' GGCTGGGATGCTGTTTGAG 3' to yield a 130 bp PCR fragment containing the A/G polymorphism. PCR reactions were performed in 25-µl reaction volume containing 60-100 ng of template DNA, GeneAmp 10x PCR buffer (Applied Biosystems), 0.2 mM of dNTP Mix (GE Healthcare Biosciences Ab), 0.4 µM of each primer, and 1.5 U of AmpliTaq polymerase (AB). The following PCR cycles were used for amplification: 94°C for 3 minutes, 35 cycles of 94°C for 30 seconds denaturation, annealing temperature of 56°C for 30 seconds, and 72°C for 45 seconds extension. Final extension was 72°C for 5 minutes. The PCR product was subsequently purified by Exonuclease I (10 U/µl) and SAP (Shrimp alkaline phosphatase, 2 U/µl) (ExoSAP-IT, Amersham Biosciences) according to the manufacturer's instructions.

PCR Extension was performed using a fluorescently labeled extension primer 5' GATGCTGTTTGAGCGCATCATGCTGGGCCC 3' and a nucleotide mix containing the stopping nucleotide ddCTP in place of the normal cytosin. PCR Extensions were performed in 20-µl reaction volume containing 2 µl of purified PCR product, Thermo Sequenase Reaction Buffer (GE Healthcare Biosciences Ab), 50 µM of each dATP, dGTP, dTTP, and ddCTP (GE Healthcare Biosciences Ab), 0.2 µM of SNuPE primer, and 6,4 U of Thermo Sequenase DNA Polymerase (GE Healthcare Biosciences Ab). The following PCR cycles were used for extension reactions: 95°C for 2 minutes, 25 cycles of 95°C for 20 seconds denaturation, annealing temperature of 56°C for 20 seconds, and 70°C for 40 seconds extension. Final extension was 70°C for 10 minutes. This yielded extension products of: 43 bp and 49 bp depending on whether G or A is present in the template. The products of the primer extension reaction were run on the ABI3730 sequencer/genotyper and results analysed using GeneMapper v3 software (Applied Biosystems).

### c) Interpretation of LOH results

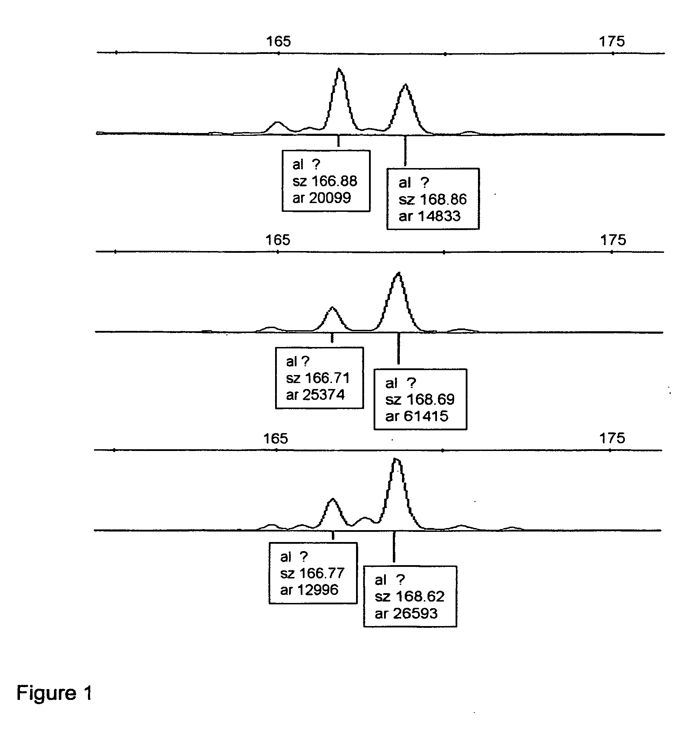

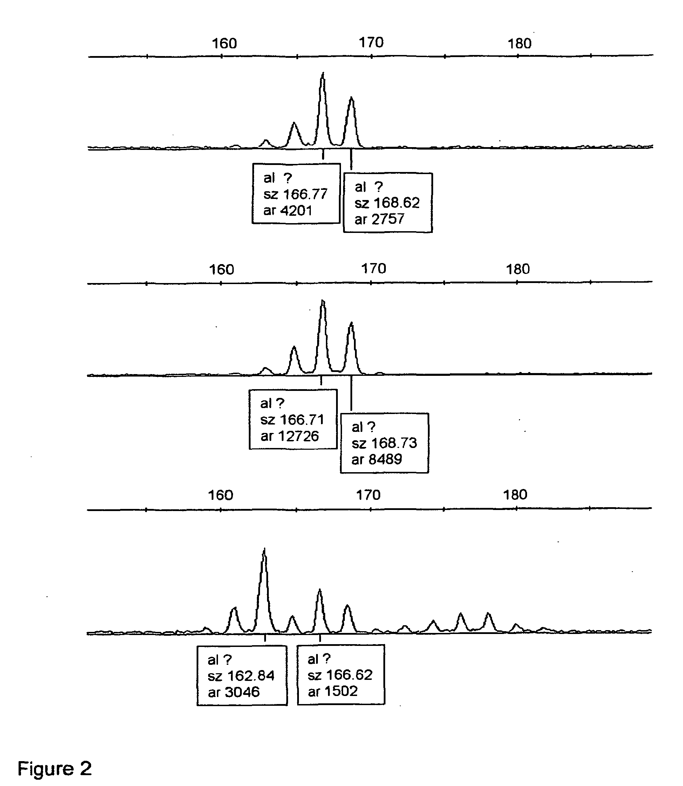

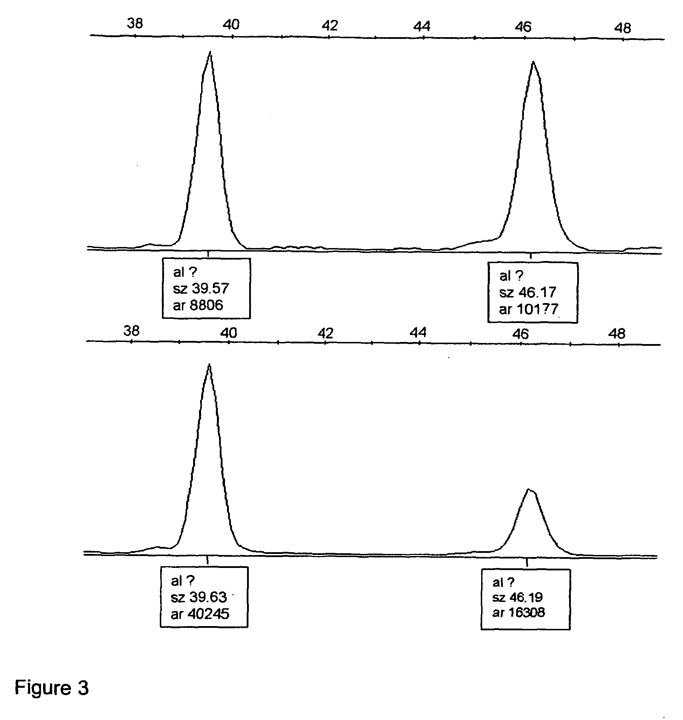

A sample was scored as showing LOH, if one of the alleles in the tumor sample had 40% or more decreased signal compared to its matching normal. There is a great consensus in the literature for using this cut off level as it is specific and sensitive enough if the tumor percentages are more than 50%, which was indeed the minimum in this study. Signal reduction up to 23% was observed in normal tissues (e.g. Cleton-Jansen et al, Cancer Res 61:1171, 2001) leaving a gray zone between 25%-39% signal reduction. These were considered here as borderline LOH "BLOH" in accordance with the published literature (Cleton-Jansen et al, Cancer Res 61:1171, 2001, Vauhkonen et al. Gastric Cancer 8: 238-244, 2005 and Kim et al. Virchows Arch 443: 491-500, 2003) (Figures 1-3).

### Example 2

### a) NAV3 LOH analysis of colorectal tumor series

Three series (designated here A, B and C) were examined:
Series A: Consecutive series of 56 colorectal carcinomas and 21 adenomas (total no = 77) from 59 patients. Adenomas and carcinomas arising in the same patients were available in 10 out of the 59 cases. Judged only by the instability at the three chromosome 12 microsatellite loci examined, all of the adenomas were MSS while 14 of the 56 carcinomas showed MSI at one or more marker(s) (25%).
Series B: Well-characterized series of familial colorectal tumors that tested negative for mismatch repair gene germline mutations. This consisted of 18 MSS carcinomas, 1 MSI carcinoma and 4 MSS adenomas (total number = 23 tumors). This series has previously been characterized for the common molecular changes in colorectal carcinogenesis.
Series C: Well-characterized series of MSI-colorectal cancers arising in HNPCC families with proven MMR gene germline mutations (total number = 24 tumors).

Corresponding normal samples were mostly from normal mucosae blocks or, when these were not available, from other normal body tissues available from the patients (eg. lymph nodes, appendix or blood).

Microsatellite instability, MSI, refers to genome-wide length variation of microsatellites, which are short tandem nucleotide tracts within the DNA, as a result of a failure in DNA mismatch repair, whereas microsatellite stable, MSS, refers to constant length of microsatellites, in other words lack of length variation of microsatellites caused by a failure in DNA mismatch repair.

The LOH analysis was performed as described in Example 1.

The results obtained from all series examined are summarized in Table 1. Table 2 gives the results for each marker tested separately. Of special note that, using the intragenic *NAV3* SNP rs1852464 as a strictly specific marker for LOH at *NA V3* exon 19, the available data so far shows a frequency of 7/20 (35%) for *NAV3* loss at this site in MSS tumors. These 7 tumors included 6 already implicated by the microsatellite markers while in only one case BLOH was seen at the rs1852464 SNP but not at the flanking microsatellite loci. Out of the 31 tumors that showed LOH/BLOH by microsatellite markers in series A, 23 were non informative or are still pending, leaving 8 cases for comparison with SNuPE. Of these 8 cases, 6 showed concordant *NAV3 loss* at the SNP rs1852464.

**Table 1. NAV3 LOH and BLOH results in different series**

| | **MSS carcinoma** | **MSI carcinoma** | **MSS adenoma** |
|---|---|---|---|
| **Series A (n=77)*** | 22/42 (52%) | 1/8 (13%)* | 8/21 (38%) |
| **Series B (n=23)** | 12/18 (67%) | 0/1 (0%) | 3/4 (75%) |
| **Series C (n=24)** | | 5/24 (21%) | |
| **Total** | 34/60 (57%) | 6/33 (18%) | 11/25 (44%) |

| | | | |
|---|---|---|---|
| * The MSI carcinoma in Series A consisted of 14 cancer of which 6 were non informative | | | |

**Table 2. NAV3 LOH and BLOH results for each marker**

| | **D12S1684** | **D12S326** | **D12S1708** | **SnuPE @rs1852464** |
|---|---|---|---|---|
| A-MSS carcinoma | 14/37 | 13/33 | 7/29 | 9/23* |
| A-MSI carcinoma | 0/3 | 0/2 | 1/4 | 0/7* |
| A-MSS adenoma | 4/21 | 6/19 | 3/16 | 0/8* |
| B-MSS carcinoma | 6/13 | 6/12 | 2/10 | 4/8 |
| B-MSI carcinoma | 0/1 | 0/1 | 0/1 | 1/1 |
| B-MSS adenoma | 1/4 | 1/2 | 1/3 | 0/1 |
| C-MSI carcinoma | 3/5 | 0/6 | 2/11 | 0/15 |
| **Totals:** | | | | |
| All MSS carcinoma | 20/50 (40%) | 19/45 (42%) | 9/39 (23%) | 13/31 (42%) |
| All MSI carcinoma | 3/9 (33%) | 0/9 (0%) | 3/16 (19%) | 1/23 (4%) |
| All MSS adenoma | 5/25 (20%) | 7/21 (33%) | 4/19 (21%) | 0/9 (0%) |
| **Total all tumors** | **28/84 (33%)** | **26/75 (35%)** | **16/74 (22%)** | **14/63 (22%)** |

| | | | | |
|---|---|---|---|---|
| * NOTE: As usual LOH frequencies were calculated for informative cases only. SNuPE test was uninformative due to constitutional homozygosity in 9 carcinomas (8 MSS, 1 MSI) and in all 6 adenomas that showed LOH by chromosome 12 microsatellites examined. This chance occurrence of homozygosity at the rs1852464 will make it risky to compare the available data by microsatellites versus SNuPE. | | | | |

### 2b) LOH analysis of colorectal adenomas and carcinomas arising in the same patient

Adenomas and carcinomas arising in the same patients were available in 10 out of the 59 cases in series A. The analysis was performed as described in Example 1.

In 4 of these 10 patients the adenomas showed LOH or BLOH and in 3 of these 4 cases the matching carcinomas were informative and have mostly similar pattern of LOH/BLOH (Table 3). This suggests that when *NAV3* LOH is observed in adenomas it is likely that such patient will develop carcinomas through *NAV3* LOH as well.

**Table 3. LOH results of adenomas and carcinomas arising in the same patient**

| **Case no** | **Tumor** | **D12S1684** | **D12S326** | **D12S1708** | **SnuPE @rs1852464** |
|---|---|---|---|---|---|
| 1 | Ca | BLOH (0.73) | no | no | |
| | Ad | BLOH (0.67) | no | no | |
| | Ad | ND | BLOH (0.74) | LOH | |
| 2 | Ca | no | LOH | no | |
| | Ad | no | BLOH (0.68) | no | |
| | Ad | no | LOH | no | |
| 3 | Ca | no | no | no | |
| | Ad | no | no | no | |
| | Ad | no | no | no | |
| 4 | Ca | BLOH (0.75) | homozygous | no | |
| | Ad | no | homozygous | no | |
| 5 | Ca | MSI | MSI | MSI | homozygous |
| | Ad | no | no | no | |
| 6 | Ca | no | no | BLOH (0.66) | |
| | Ca | no | no | BLOH (0.66) | |
| | Ad | no | no | no | |
| 7 | Ca | no | no | homozygous | |
| | Ad | no | no | homozygous | |
| 8 | Ca | no | homozygous | homozygous | |
| | Ad | no | homozygous | homozygous | |
| 9 | Ca | MSI | MSI | ?MSI | homozygous |
| | Ad | BLOH (0.73) | no | homozygous | |
| 10 | Ca | LOH | LOH | LOH | |
| | Ad | LOH | LOH | LOH | |

### 2c) Morphological and histological features of colorectal tumors carrying NAV3 LOH

The occurrence of LOH in the adenomas tended to be associated to cases, which by standard criteria were considered to be at risk to cancer development. Out of five adenomas with LOH, the mean diameter was more than 9 mm and thus, close to the critical level of 1 cm, while in the other five cases without LOH, the mean diameter was less than 6 mm (Table 4). Furthermore, in the adenomas with LOH, one out of five showed poor differentiation while the differentiation degree in the LOH negative cases was always high.

**Table 4. LOH analysis with markers D12S1684, D12S326 and D12S1708 of ten cases with tubular adenomas of colon and rectum. Differentiation was graded from 1 (highly differentiated) to 3 (poor differentiation). Size was given as the diameter in millimeters.**

| **Case** | **D12S1684** | **D12S326** | **D12S1708** | **Differentiation*** | **Size**** |
|---|---|---|---|---|---|
| 1 | Complete LOH | LOH | LOH | 1 | 15 |
| 2 | No | BLOH | No | 2 | 10 |
| 3 | No | BLOH | No | 1 | 8 |
| 4 | BLOH | No | No | 1 | 7 |
| 5 | No | LOH | No | 1 | 7 |
| 6 | No | No | No | 1 | 7 |
| 7 | No | No | No | 1 | 6 |
| 8 | No | Homozygous | Homozygous | 1 | 5 |
| 9 | No | No | No | 1 | 5 |
| 10 | No | No | No | 1 | 5 |

| | | | | | |
|---|---|---|---|---|---|
| * Differentiation degree 1-3 ** Size in millimeters | | | | | |

### Example 3

### NAV3 deletion in colorectal tumors detected by FISH

### Samples

Samples for the FISH (fluorescence in-situ hybridization) assay were prepared from 18 randomly selected colorectal carcinoma cases from series A (described in Example 2a) and from seven cases with skin samples obtained from patients suffering from chronic eczema, a non-malignant inflammatory lesion as a negative control. All tissue samples had been processed by routine formalin fixation and embedded in paraffin.

### Preparation of nuclei from paraffin embedded tissue

50 µm sections were cut from formalin-fixed paraffin embedded tissue. After deparaffinization each section was digested with protease XXIV (Sigma) at +37°C for 30 minutes. After enzymatic digestion, nuclei were pelleted by centrifugation at 2000 g for 10 minutes and diluted in 0.1 M Tris-HCl, 0.07 M NaCl, pH 7.2. Nuclear suspension was pipetted on objective slides and dried over night at room temperature. The slides were fixed with 0.01% paraformaldehyde for 4 minutes at room temperature, followed by dehydration with graded ethanol (70%, 85%, 100%). Slides were stored at -70°C.

### Labeling of probes with Fluorescein-12-dUTP and with Alexa-594-5-dUTP

Three bacterial artificial chromosome (BAC) clones specific to *NAV3* DNA (RP11-494K17, RP11-36P3 and RP11-136F16; Research Genetics Inc., Huntsville, AL, USA) were labeled with Alexa-594-5-dUTP (Invitrogen) and the chromosome 12 centromere probe (pA12H8) was labeled with Fluorescein-12-dUTP (Roche) using nick translation (Hyytinen E et al. Cytometry, 16: 93-99, 1994). For each labeling reaction 1-2 µg DNA was used in total reaction volume of 50 µl. 4 µl of each labeled BAC and centromere probes were mixed together with human COT1 DNA (Invitrogen) and precipitated with sodium acetate and ethanol. Precipitated probe mix was diluted into hybridization buffer (15% w/v dextran sulphate, 70% formamide in SSC, pH 7.0) and denatured at +76°C for 10 minutes.

### FISH with nuclei extracted from paraffin embedded tissue

Slides were pretreated with 1 M sodium thiocyanate at +80°C for 5 minutes and washed with 2 x SSC three times for 5 minutes. After washing slides were treated with 50% glycerol, 0.1 x SSC at +90°C for 6 minutes, with 2 x SSC for 3 minutes and with distilled water three times for 2 minutes. Slides were denatured in 70% formamide, 2 x SSC at +87°C for 7 minutes. After denaturation the slides were dehydrated with graded ethanol (70%, 85%, 100%) and digested enzymatically with proteinase K (Sigma; 8 µg/ml in 20 mM Tris-HCl, pH 7.5, 2 mM CaCl₂) at +37°C for 7 minutes. After digestion slides were dehydrated and 10 µl of denatured probe mix was pipetted on slides. Hybridisation was carried out overnight at +37°C. Slides were washed three times with 1.5 M Urea, 0.1 x SSC at +45°C for 10 minutes, once with 0.1 x SSC for 10 minutes and 4 x SSC for 5 minutes, followed by three washes with PN buffer (0.1 M sodium phosphate buffer, pH 8.0, 0.1% NP-40). Finally, slides were rinsed with distilled water, air dried and mounted in Vectashield Mounting Medium with DAPI (Vector).

### Analysis and results

Slides were analysed using Olympus BX 50, Tokyo, Japan, equipped with filter set 8300 and tripleband exciter 83103x (Chroma Technology Corp., Brattleboro, VT, USA) and a cooled CCD camera (Sensi Cam, PCO, Computer Optics, Kelheim, Germany) combined to a computer (Dell GX280, Limerick, Ireland) with software Image pro Plus (Media Cybernetics, Silver Spring, MD, USA). Fifty cells were analysed from each case and the cells were grouped as normal if having two labels for chromosome 12 centromere and two for the *NAV3.* Polyploid cells had three or more centromere labels. *NAV3* deletion was defined when the number of centromere labels was higher than the number of *NAV3* labels. A few cells had one centromere and one *NAV3* label; this was taken as a technical artifact. The results (Table 5) show clearly that the samples from the colon carcinomas have a high frequency of polyploidy and that these cells often show deletion of one or more of the *NAV3* alleles.

**Table 5. Number of normal cells, polyploidy cells and cells with NAV3 deletion in samples from colorectal carsinoma patients and from inflammatory skin lesions. Fifty cells per case were calculated.**

| Sample | Number of cells counted | normal 2/2 cell, * | all polyploid cells (>2 cen), ** | cen>nav cells, *** | 1 cen 1 nav, **** |
|---|---|---|---|---|---|
| Case C1, colon carsinoma | 50 | 28 | 14 | 5 | 2 |
| Case C2, colon carsinoma | 50 | 31 | 6 | 11 | 3 |
| Case C3, colon carsinoma | 50 | 29 | 10 | 8 | 7 |
| Case C4, colon carsinoma | 50 | 27 | 17 | 9 | 2 |
| Case C5, colon carsinoma | 50 | 22 | 15 | 20 | 2 |
| Case C6, colon carsinoma | 50 | 19 | 18 | 7 | 5 |
| Case C7, colon carsinoma | 50 | 31 | 5 | 6 | 7 |
| Case C8, colon carsinoma | 50 | 21 | 21 | 11 | 5 |
| Case C9, colon carcinoma | 50 | 16 | 30 | 10 | 2 |
| Case C10, colon carsinoma | 50 | 18 | 16 | 16 | 7 |
| Case C11, colon carsinoma | 50 | 19 | 18 | 8 | 4 |
| Case C12, colon carsinoma | 50 | 23 | 18 | 10 | 0 |
| Case C13, colon carsinoma | 50 | 34 | 1 | 3 | 5 |
| Case C14, colon carsinoma | 50 | 19 | 18 | 8 | 4 |
| Case C15, colon carsinoma | 50 | 23 | 18 | 10 | 5 |
| Case C16, colon carsinoma | 50 | 24 | 11 | 8 | 3 |
| Case C17, colon carsinoma | 50 | 28 | 12 | 6 | 3 |
| Case C18, colon carsinoma | 50 | 24 | 14 | 12 | 3 |
| | | | | | |
| Case S1, skin, eczema | 50 | 42 | 1 | 2 | 3 |
| Case S2, skin, eczema | 50 | 43 | 1 | 3 | 3 |
| Case S3, skin, eczema | 50 | 47 | 1 | 0 | 2 |
| Case S4, skin, eczema | 50 | 42 | 0 | 2 | 5 |
| Case S5, skin, eczema | 50 | 44 | 1 | 3 | 2 |
| Case S6, skin, eczema | 50 | 38 | 3 | 3 | 9 |
| Case S7, skin, eczema | 50 | 41 | 2 | 2 | 3 |

| | | | | | |
|---|---|---|---|---|---|
| * = Normal number (2 and 2) of centromere and *NAV3* labels per cell ** = More than 2 centromere labels per cell *** = Number of centromere labels more than *NAV3 labels* per cell **** = one centromere and one *NAV3* label per cell | | | | | |

### Example 4

### NAV3 LOH analysis of lung tumors

Archival paraffin-embedded samples of five patients with lung cancer without any evidence of other organ involvement or other concomitant cancer were examined. Three of the lung cancer samples were SCLC and two epidermoid carcinomas. Microdissection and PCR amplification for the lung cancer cell samples and their corresponding normal lung tissue samples were performed according to the following protocol.

Sections of 5-µm were cut from the samples using a microtome and mounted onto a 1,35 µm thin polyethylene membrane (P.A.L.M. Microlaser Technologies, Bemried, Germany) attached to a glass slide. Tissue sections were then deparaffinized and stained with hematoxylin as described before (Stoecklein et al. Am J Pathol 161: 43-51, 2002). For morphological control hematoxylin-eosin staining was made according to standard protocol. Areas of malignant cells covering 200000 µm² were laser capture microdissected using the P.A.L.M. Laser-Microbeam system (P.A.L.M. Microlaser Technologies). Thereafter, proteinase K digestion was performed and the DNA was amplified with SCOMP as previously described (Klein et al. Proc Natl Acad Sci USA 96: 4494-9, 1999 and Stoecklein et al. Am J Pathol 161: 43-51, 2002). The success of amplification was PCR-tested for microsatellite markers D5S500 and D17S1161, as previously described (Klein et al. Proc Natl Acad Sci USA 96: 4494-9, 1999 and Stoecklein et al. Am J Pathol 161: 43-51, 2002).

The five lung cancer cases were analysed successfully for LOH according to the method described in Example 1 a. Of these five lung cancers, one was uninformative for all three markers that were used, but loss of heterozygosity was found in two of the four other cases (Table 6).

**Table 6. NAV3 LOH in lung cancers.**

| **Case** | **D12S326 (closer to *NAV3)*** | **D12S1708** | **D12S1684** |
|---|---|---|---|
| EC normal | OK-heterozygous | homozygous | ?? |
| EC tumor | NO | Homozygous | ?? |
| SCLC normal | OK-heterozygous | homozygous | homozygous |
| SCLC tumor | LOH | ?? | ?? |
| EC normal | OK-heterozygous | OK-heterozygous | OK-heterozygous |
| EC tumor | NO | LOH | LOH |
| SCLC normal | Homozygous | homozygous | homozygous |
| SCLC tumor | homozygous | homozygous | ?? |
| SCLC normal | homozygous | OK-heterozygous | homozygous |
| SCLC tumor | homozygous | NO | homozygous |

| | | | |
|---|---|---|---|
| ?? means non-interpretable pattern NO = no LOH SCLC = small cell lung cancer EC = epidermoid carcinoma of the lung | | | |

### Example 5

### CGH-analysis of lung tumors

Lung cancer samples of twelve patients were used for CGH. These twelve patients were also diagnosed with CTCL.

CGH was performed according to the protocol published by Klein *et al.* 1999 with the modifications described by Stoecklein *et al.* 2002 (Klein et al. Proc Natl Acad Sci USA 96: 4494-9, 1999 and Stoecklein et al. Am J Pathol 161: 43-51, 2002). Briefly, microdissected and proteinase K digested DNA was digested with Mse I restriction enzyme (BioLabs) resulting in DNA fragments with an average length of 256 bp, adaptors were ligated to the 5' overhangs, and DNA fragments were amplified by polymerase chain reaction. The amplified DNA was then labeled with digoxigenin-dUTP (Roche) and similarly processed aliquots of reference DNA obtained from peripheral blood mononuclear cells of healthy volunteers with biotin-dUTP (Roche). The labeled probes were hybridized on normal male metaphase slides for 2-3 nights. After posthybridization washes, metaphases were viewed under a fluorescence microscope and three-color digital images were captured using an epifluorescence microscope (Axioplan imagining 2, Carl Zeiss AG, Oberkochen, Germany) equipped with a CCD camera using statistical limits for green to red ratios to determine DNA copy number gains and losses. Eight to twelve metaphases were included in the analysis for each case. As an internal control, normal male and female DNA were cohybridized and only differences in sex chromosomes were identified.

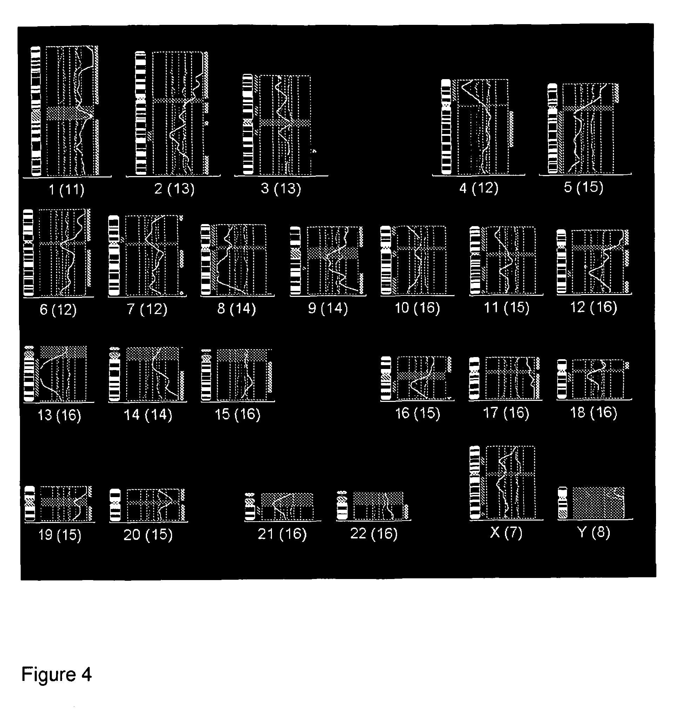

In tumor cells of one lung cancer patient, loss of 12q21 was shown by CGH (Figure 4). Karyotype of the patient with SCLC and CTCL represent typical changes for SCLC: losses of 3p, 5q, 8p, 10q, and 13q, as well as gains of 5p, and 19q. Some other typical SCLC aberrations (17p loss, and 8q gain) are absent. Findings characteristic for CTCL include e.g. losses of 10q/10, and 13, and gains of 4q, 7, 17q/17, and 18, which all can be demonstrated in this case. Interestingly, loss of 12q21 was also evident.

### Example 6

### NAV3 deletions in urinary bladder cancer

### Tissue samples

Samples from 16 patients diagnosed to have a transitional epithelial carcinoma of the urinary bladder were selected for the study. The samples were routinely fixed in neutral formalin and embedded in paraffin. 1-3 sections of 50 microns thickness were cut and the nuclei were isolated as described in Example 3, page 18, second paragraph.

### Probe labeling

Two bacterial artificial chromosome (BAC) clones specific to *NAV3* DNA (RP11-36P3 and RP11-136F16; Research Genetics Inc., Huntsville, AL, USA) were labeled with Alexa594-5-dUTP (Invitrogen) and the chromosome 12 centromere probe (pA12H8; American Type Cell Culture) was labeled with Alexa488-5-dUTP (Invitrogen) using nick translation (Hyytinen et al. 1994). 50-75 ng of each labeled BAC and 30 ng of centromere probe were mixed together with 1 µg of human COT1 DNA (Invitrogen) and precipitated with sodium acetate and ethanol. Precipitated probe mix was diluted into 10 µl of hybridization buffer (15% w/v dextran sulphate, 70% formamide in 2 x SSC, pH 7.0).

### Fluoresence in situ hybridisation

Nuclei slides were pretreated with 1 M sodium thiocyanate at +80°C for 5 minutes and washed with 2 x SSC three times for 5 minutes at room temperature. After washing, slides were treated with 50% glycerol, 0.1 x SSC at +90°C for 6 minutes, with 2 x SSC for 3 minutes and with distilled water three times for 2 minutes. Slides were digested enzymatically with proteinase K (Sigma; 8µg/ml in 20 mM Tris-HCl, pH 7.5, 2 mM CaCl₂) at +37°C for 8 minutes. After dehydration and air drying probe mix was pipetted on slides and slides were denatured for 6 min at +85°C on a hot plate. Hybridisation was carried out for 48 hr at +37°C. Slides were washed three times with 1.5 M Urea, 0.1 x SSC at +47°C for 10 minutes, once with 0.1 x SSC for 10 minutes at +47°C, followed by three washes with PBS, 0.1 % NP-40 at room temperature. Finally, slides were rinsed with distilled water, air dried and mounted in Vectashield Mounting Medium with 4',6-diamino-2 phenylindole dihydrochloride (DAPI; Vector).

FISH results were evaluated using Olympus BX51 microscope (Tokyo, Japan) equipped with a 60X oil immersion objective and a triple bandpass filter for simultaneous detection of Alexa488, Alexa594 and DAPI (Chroma Technology Corp., Brattleboro, VT, USA). 200 nuclei were analysed from each case and the nuclei were grouped as normal if having two labels for chromosome 12 centromere and two for the *NAV3.* Polyploid nuclei had three or more centromere labels. *NAV3* deletion was defined when the number of centromere labels was higher than the number of *NAV3* labels and *NAV3* amplification was defined when the number of *NAV3* labels was higher than centromere labels. The analyses were done blinded to the diagnosis or sample identity by two independent analysers.

### NAV3 LOH analysis using microsatellite markers

For LOH assay (loss of heterozygocity), DNA coming both from normal tissue of the patient as well as from the tumor samples was extracted from the 10 µm thick paraffin embedded sections following standard methods (Isola et al. Am J pathol 145: 1301-1308, 1994). Analysis was performed as described in Example 1a.

### Results

The results concerning the LOH (and borderline LOH; BLOH) as well as the status of *NAV3* copy number in the FISH assay are shown in Table 7. 7 out of 17 urinary bladder cancer samples (40%) showed LOH/BLOH with at least one of the markers used in the study. 20 out of 64 (30%) alleles were homozygous and could not be analyzed using LOH method. In FISH analysis, 3 out of 15 samples (20%) showed *NAV3* deletion. *NAV3* gene duplication (amplification) was seen with 20% of samples. One of the samples had both *NAV3* deletion and amplification. Two samples were not analyzed for *NAV3* copy number changes due to poor quality of the sample.

**Table 7. NAV3 LOH and FISH analysis results in urinary bladder cancer**

| **Case no** | **D12S1684** | **D12S326** | **D12S1708** | **SnuPE@ rs1852464** | **NAV3 aberration (by FISH)** |
|---|---|---|---|---|---|
| 1 | No | No | No | No | - |
| 2 | BLOH | LOH | Homozygous | Homozygous | deletion |
| 3 | No | BLOH | No | No | - |
| 4 | No | BLOH | No | Homozygous | deletion |
| 5 | No | No | No | No | deletion and amplification |
| 6 | BLOH | BLOH | No | No | - |
| 7 | No | Homozygous | No | Homozygous | amplification |
| 8 | No | Homozygous | Homozygous | Homozygous | NA |
| 9a | No | No | No | BLOH | - |
| 9b | No | LOH | No | No | amplification |
| 10 | BLOH | BLOH | BLOH | Homozygous | deletion |
| 11 | No | No | No | Homozygous | - |
| 12 | No | Homozvqous | Homozygous | Homozygous | - |
| 13 | Homozygous | No | Homozygous | Homozygous | - |
| 14 | No | No | Homozygous | No | - |
| 15 | No | Homozygous | Homozygous | No | NA |
| 16 | No | No | No | Homozygous | amplification |
| **No of postive findings** | **3/17, 1 non informative** | **6/17, 4 non informative** | **1/17, 6 non informative** | **1/17, 9 non informative** | **7/15, 2 samples not analysed (NA)** |

### Example 7

### NAV3 deletion in breast cancer

### Tissue samples

We studied the occurrence of *NAV3* deletions in breast cancer by selecting sentinel lymph nodes from four patients operated upon for breast cancer as a study material. Touch preparates were performed from freshly obtained lymph nodes or from frozen material and stored at -70°C until used for *NAV3* FISH analysis.

### Probe labeling

Two bacterial artificial chromosome (BAC) clones specific to *NAV3* DNA (RP11-36P3 and RP11-136F16; Research Genetics Inc., Huntsville, AL, USA) were labeled with Alexa594-5-dUTP (Invitrogen) and the chromosome 12 centromere probe (pA12H8; American Type Cell Culture) was labeled with Alexa488-5-dUTP (Invitrogen) using nick translation (Hyytinen et al. 1994). 50-75 ng of each labeled BAC and 10 ng of centromere probe were mixed together with 1 µg of human COT1 DNA (Invitrogen) and precipitated with sodium acetate and ethanol. Precipitated probe mix was diluted into 10 µl of hybridization buffer (15% w/v dextran sulphate, 70% formamide in 2 x SSC, pH 7.0).

### Fluorescence in situ hybridisation

Slides were fixed with 4% paraformaldehyde in PBS for 1 minute on ice. After PBS washes, slides were digested enzymatically with proteinase K (Sigma; 0.66 µg/ml in 20 mM Tris-HCl, pH 7.5, 2 mM CaCl₂) at +37°C for 6 minutes. After dehydration and air drying probe mix was pipetted on slides and slides were denatured for 5 min at +75°C on a hot plate. Hybridisation was carried out for 24 hr at +37°C. Slides were washed three times with 1.5 M Urea, 0.1 x SSC at +47°C for 10 minutes, once with 0.1 x SSC for 10 minutes at +47°C, followed by three washes with PBS, 0.1% NP-40 at room temperature. Finally, slides were rinsed with distilled water, air dried and mounted in Vectashield Mounting Medium with 4',6-diamino-2 phenylindole dihydrochloride (DAPI; Vector).

FISH results were evaluated using Olympus BX51 microscope (Tokyo, Japan) equipped with a 60X oil immersion objective and a triple bandpass filter for simultaneous detection of Alexa488, Alexa594 and DAPI (Chroma Technology Corp., Brattleboro, VT, USA). All cancer cells found from the sample were analysed from each case and the cells were grouped as normal if having two labels for chromosome 12 centromere and two for the *NAV3.* Polyploid cells had three or more centromere labels. *NAV3* deletion was defined when the number of centromere labels was higher than the number of *NAV3* labels and *NAV3* amplification was defined when the number of *NAV3* labels was higher than centromere labels. The analyses were done blinded to the diagnosis or sample identity by two independent analyzers.

### Results

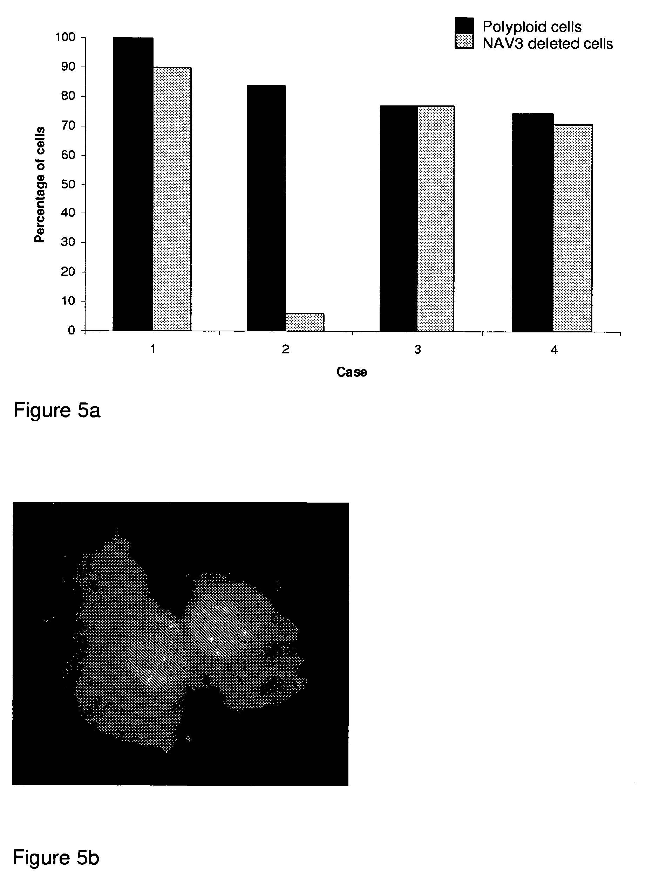

The results are shown in table 8 and Figures 5a and 5b. Four cases were analyzed and all cancer cells found from the samples were counted from each case. Cells were grouped as normal if having two labels for chromosome 12 centromere and two for the *NAV3.* Polyploid cells had three or more centromere labels (>2cen). *NAV3* deleted cells contained higher number of centromere labels than *NAV3* labels (cen>nav) and *NAV3* amplified cells had higher number of *NAV3 labels* than centromere labels (cen<nav).

All four cases showed cells that contained abnormal copy numbers of centromeres and/or *NAV3.* In addition to *NAV3* deletion (less than two copies of the *NAV3* signal in FISH), number of cells with polyploidy (more than two copies of chromosome 12 centromere) and *NAV3* amplification (more than two copies of *NAV3* signal) was analyzed and recorded. In one case (case number 2) polyploidy with less extensive *NAV3* deletion was observed, while in the remaining three cases polyploidy was associated with the loss of a *NAV3* allele.

In Figure 5a, black bars indicate the amount of polyploidy in studied cells and grey bars indicate the amount of *NAV3* deleted cells. Results are shown as percentage of total cell count. In Figure 5b, typical cancer cells show *NAV3* deletion. Green signals indicate centomeres and red signals *NAV3* copies.

**Table 8. NAV3 FISH results with breast cancer samples**

| **Case** | **1** | **2** | **3** | **4** |
|---|---|---|---|---|
| **No of counted cancer cells** | 20 | 69 | 52 | 55 |
| **% Normal cells** | 0 | 14.5 | 19 | 23.5 |
| **% Polyploid cells** | 100 | 84 | 77 | 74.5 |
| ***% NAV3* amplified cells** | 10 | 6 | 0 | 2 |
| ***% NAV3* deleted cells** | 90 | 6 | 77 | 71 |
| **Primary cell type (cen+NAV3)** | 4+2 | 4+4 | 4+2 | 4+2 |

It is noteworthy that while it was difficult or even impossible to find the malignant breast cancer cells in the lymph node touch preparate using just routine light microscopy, this task became very simple after the *NAV3* alleles were marked with the specific fluorescence probe. Even single cells, in the middle of thousands of normal lymphocytes, could be identified with clear copy number changes and as a rule, these cells also showed the characteristic atypical nuclei of cancer cell. Such abnormal cells would have been extremely difficult to identify with light microscopy.

### Example 8

### NAV3 copy number changes in basal cell carcinoma (BCC) and squamous cell carcinoma (SCC)

### Tissue samples

Samples from 14 patients diagnosed to have a basal cell carcinoma and 5 patients with squamous cell carcinoma were selected for the study. The samples were routinely fixed in neutral formalin and embedded in paraffin. 1-3 sections of 50 microns thickness were cut and the nuclei were isolated as described in Example 3, page 18, second paragraph.

### Fluorescence in situ hybridization

Probe labeling and FISH analysis were performed as described in example 6.

### Results

*NAV3* FISH analysis results of BCC and SCC samples are shown in Table 9. 3 out of 14 (21 %) of the BCC samples showed *NAV3* deletion with deletion range of 6-11 % of the total cell count. In addition, three of the samples (21 %) showed *NAV3* gene duplication (amplification range 8-11 %). One out of five (20%) of SCC samples indicated *NAV3* deletion (12%).

**Table 9. NAV3 FISH results with BCC and SCC samples**

| **Case** | **Normal cells (% of total cell count)** | **Polyploid cells (% of total cell count)** | ***NAV3* amplified cells (% of total cell count)** | ***NAV3* deleted cells (% of total cell count)** |
|---|---|---|---|---|
| BCC1 | 87 | 3 | NA | 2 |
| BCC2 | 87 | 4 | NA | 4 |
| BCC3 | 77 | 14 | 9 | 4 |
| BCC4 | 85 | 4 | 5 | 7 |
| BCC5 | 84 | 3 | 8 | 4 |
| BCC6 | 90 | 2 | 3 | 4 |
| BCC7 | 85 | 7 | 4 | 2 |
| BCC8 | 75 | 20 | 5 | 3 |
| BCC9 | 88 | 2 | 7 | 1 |
| BCC10 | NA | 2 | NA | 1 |
| BCC11 | NA | NA | NA | 1 |
| BCC12 | NA | NA | NA | 1 |
| BCC13 | NA | 17 | 5 | 11 |
| BCC14 | NA | 7 | 11 | 6 |
| SCC1 | 89 | 4 | 4 | 2 |
| SCC2 | 90 | 6 | 3 | 2 |
| SCC3 | 94 | 2 | 2 | 2 |
| SCC4 | 81 | 4 | NA | 3 |
| SCC5 | NA | 9 | NA | 12 |

### Example 9

### NAV3 copy number changes in colon cancer

### Samples and NAV3 FISH analysis

a) Two MSS-type colorectal adenocarcinoma cell lines [CCL-230 (SW403) and CCL-228 (SW480)] and two normal colon cell lines [CRL-1539 (CCD-33Co) and CRL-1541 (CCD-112CoN)] were ordered from American Type Culture Collection (LGC Promochem AB, Boras, Sweden) and grown at +37°C following manufacturer's instructions. 50 000-100 000 cells were spun down onto the Super Frost Plus slide using cytocentrifuge. Slides were air-dried, fixed with acetone and stored at -70°C until used for *NAV3* FISH analysis. Probe labeling and FISH analysis were similar to breast cancer samples in Example 7.
**b**) For metaphase preparations colorectal adenocarcinoma cell lines CCL-248, SW403, SW480, RKO, DLD, HCA7, LIM1215 and LOVO (American Type Culture Collection, LGC Promochem AB, Boras, Sweden) were grown following ATCC's instructions.
   Cells were treated with hypotonic KCI-solution, fixed with acetone-methanol (1:3) and the cell suspension was dropped on objective slides to make conventional chromosome preparations.
   Purified DNA of pA12H8 (centromere 12, plasmid from ATTC, purified as above or according to Karenko L et al. J Invest Dermatol 108: 22-29, 1997) and purified DNA of RP11-136F16 and BAC RP11-36P3 (Karenko L et al. Cancer Res 65: 8101-8110, 2005), were labelled with nick-translation with FITC-dUTP (NEN Life Science products, Inc, Boston, MA US), Alexa-594-dUTP (Invitrogen Molecular Probes, Leiden, Netherlands), biotin-dATP (Gibco BRL, Gaithersburg, MD, USA) or digoxigenin-dUTP (Roche, Mannheim, Germany). Centromere probe (e.g. 1-5 ng) labelled with FITC or biotin and one or two BAC-probes were mixed and precipitated by adding 1/10 volume 3M sodium acetate and 2 x volume 100% ethanol, and centrifuged. The supernatant was discarded, the pellet was allowed to dry, after which the DNA was dissolved in a mixture consisting of 50% formamide, and 10% dextran sulphate, 2 x SSC, pH7 and optionally Cot-1 DNA (e.g. 125ng; Gibco BRL, Gaithersburg, MD, USA), called here probe mixture. Target metaphases on slides were denatured in for 2 to 3 minutes in 70% formamide / 2 x SSC solution (pH 7.0) at 70 to 73°C, and dehydrated in 70%, 85%, and 100% ethanol, and treated with proteinase K (1 µg/ml, Sigma Chemical Co, St Louis, MO, USA) in 20 mM Tris/2mM CaCl₂ (pH 7.5) buffer for 7.5 minutes at 37°C, and dehydrated as above. The probe mixture was denatured for 5 minutes in 70°C, and applied to pretreated slides on a warm plate (37°C), sealed under a coverslip with Rubber Cement (Starkey Chemical Co, LaGrange IL USA) and allowed to hybridize in a humid chamber (37°C) for 2 to 3 days. The slides were washed 3 times with 50% formamide in 2 x SSC, pH 7, 4 x SSC, and 0.1 x SSC, all at 45°C, and with 4 x SSC, 2 x SSC and PBS at room temperature. After the posthybridization washes, the biotinylated probe was visualised with avidin-FITC (green, Vector Laboratories, Burlingame, CA, USA) and the digoxigenin labeled probe was visualised with anti-digoxigenin antibody made in sheep conjugated with rhodamine (red, Roche, Mannheim, Germany). The slides were counterstained with DAPI and mounted in Vectashield (bothVector Laboratories, Burlingame, CA, USA).
   The air-dried preparations were fixed with 0.1% paraformaldehyde and dried in ethanol series (70%, 85%, 100%).
   The metaphases were photographed with UV-microscope (Axioplan imagining 2, Zeiss, Germany) and analysed using the computer program Isis of MetaSystems GmbH with MFISH-program module.
c) Samples from 36 patients diagnosed to have a MSS-type of colorectal adenocarcinoma, 14 patients with MSI-type of colorectal adenoma and 19 patients with adenoma tubulare were selected for the study. In addition, 58 normal colon mucosa samples were included in the study as a reference material. The samples were routinely fixed in neutral formalin and embedded in paraffin. 1-3 sections of 50 microns thickness were cut and the nuclei were isolated as described in Example 3. *NAV3* specific FISH assay was performed as described in Example 6.

### Results

**a)** FISH analysis results of interphase cells of colon cancer cell lines (SW 403 and SW480) are shown in table 10. Both cancer cell lines showed dominating 3 centromeres 2 NAV3 -type of deletion in almost all of the cells studied. Normal colon cell lines did not show any *NAV3* gene copy number changes.

**Table 10. NAV3 FISH results with colon cancer cell lines**

| **Cell line** | **Normal cells (2cen2NAV3) (% of cells studied)** | **Polyploid cells (cen>2) (% of cells studied)** | ***NAV3* deleted cells (cen>NAV3) (% of cells studied)** |
|---|---|---|---|
| SW403 | 5 | 95 | 92 |
| SW480 | 4 | 96 | 92 |

**b)** FISH analysis results of metaphase cells of colon cancer cell lines (CCL-248, SW 403, SW480, RKO, DLD, HCA7, LIM1215 and LOVO) are shown in table 11. *NAV3* deletion was detected in a great majority of metaphase cells in two MSS cell lines (CCL-248, SW 403). Also MSI cell line RKO showed plenty of *NAV3* deletions. Plenty of *NAV3* amplifications were detected in cell line DLD (MSI). Also line SW480 (MSS) showed more centromere signals than *NAV3* signals. Some *NA V3* deletions were detected in cell line HCA7.

**Table 11. NAV3 FISH results of metaphase cells with colon cancer cell lines**

| **Cell line** | **Type** | **Probe** | **NAV3 deleted cells (cen>NAV3) (% of cells studied)** | ***NAV3* amplified cells (cen<NAV3) (% of cells studied)** |
|---|---|---|---|---|
| CCL-248 | MSS | 36P3 | 9/10 (90%) | 0/10 (0%) |
| SW 403 | MSS | 36P3 | 6/7 (86%) | 0/7 (0%) |
| SW480 | MSS | 36P3 | 2/10 (20%) | 2/10 (20%) |
| RKO | MSI | 136F16 | 12/29 (41%) | 0/29 (0%) |
| | | 36P3 | 6/14 (43%) | 0/14 (0%) |
| | | both | 3/7 (43%) | 0/7 (0%) |
| DLD | MSI | 136F16 | 0/8 (0%) | 8/8 (100%) |
| | | 36P3 | 0/8 (0%) | 8/8 (100%) |
| | | both | 0/2 (0%) | 2/2 (100%) |
| HCA7 | MSI | 136F16 | 2/10 (20%) | 0/10 (0%) |
| | | 36P3 | 0/6 (0%) | 0/6 (0%) |
| | | both | 0/13 (0%) | 0/13 (0%) |
| LIM1215 | MSI | 136F16 | 0/2 (0%) | 0/2 (0%) |
| | | 36P3 | 0/10 (0%) | 0/10 (0%) |
| | | both | 0/15 (0%) | 0/15 (0%) |
| LOVO | MSI | both | 0/1 (0%) | 0/1 (0%) |

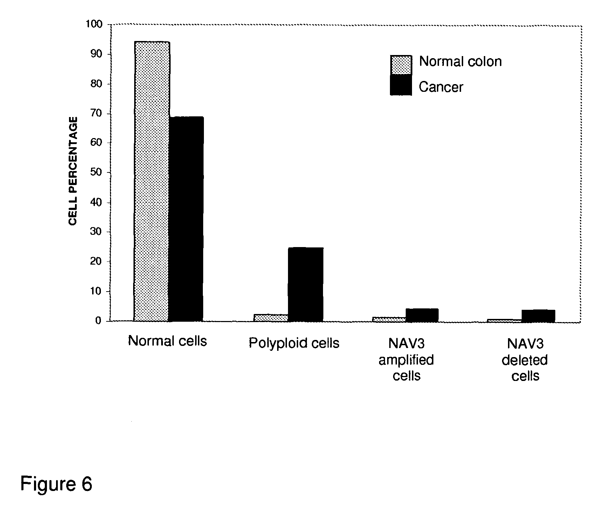

**c)** *NAV3* FISH assay with nuclei extracted from paraffin embedded patient samples indicated NAV3 copy number changes in 31% of MSS-type colorectal adenocarcinoma samples, 7% of MSI-type of colorectal adenocarcinoma (1 sample out of 14) and in 16% of adenoma tubulare samples. Results are shown in table 12. Figure 6 shows comparison of *NAV3* FISH results from normal colon samples and MSS-type of colorectal adenocarcinoma. Cancer cells are different from normal colon mucosal cells in terms of polyploidy and *NAV3* copy number.

**Table 12. NAV3 FISH analysis results using different colon samples. In each sample, 200 cells were analyzed. NAV3 deleted cells contain higher number of centromere labels than NAV3 labels and NAV3 amplified cells higher number of NAV3 labels than centromere labels.**

| **Colon sample** | **Samples with aberrant *NAV3*** | **Deletion range (% of cells studied)** | **Amplification range (% of cells studied)** |
|---|---|---|---|
| CRC, MSS | 11/36 | 5-41 | 8-28 |
| CRC, MSI | 1/14 | 7.5 | - |
| Adenoma tubulare | 3/19 | 7-11 | 8 |
| Normal colon | 0/58 | - | - |

## Claims

1. An in vitro method of predicting tumor initiation, tumor progression and/or carcinoma, **characterized by** detecting the presence or the absence of genetic abnormalities at 12q21.2 in a neuron navigator 3 *(NAV3)* gene or a fragment thereof, the presence of said genetic abnormalities indicating an initiation or progression of tumors of epithelial origin and/or carcinoma in a biological sample.

2. An in vitro method of identifying an individual with potential for developing carcinoma of epithelial origin, the method comprising detection of genetic abnormalities at 12q21.2 in a neuron navigator 3 *(NAV3)* gene or a fragment thereof, said genetic abnormalities indicating tumors of epithelial origin.

3. An in vitro method of predicting the progression of carcinomas of epithelial origin and/or the transformation thereof to an aggressive variant, **characterized by** detecting genetic abnormalities at 12q21.2 in a neuron navigator 3 *(NAV3)* gene or a fragment thereof, wherein abnormalities indicate the probability to develop carcinoma.

4. A method according to any preceding claim, **characterized in that** the tumor of epithelial origin is an adenoma and/or a carcinoma.

5. A method according to any preceding claim, **characterized in that** the tumor of epithelial origin is in colon, rectum, lung, urinary bladder, breast or in squamous or basal cells.

6. A method according to any preceding claim, **characterized in that** genetic abnormalities are determined by fluorescence *in situ* hybridization (FISH).

7. A method according to claims 1-6, **characterized by** determining the loss of heterozygosity (LOH) of *NAV3* gene or a functional fragment thereof, wherein LOH of *NAV3* is indicative of tumor progression.

8. A method according to claims 1-7 **characterized in that** genetic abnormalities *of NAV3* gene are determined in haploid, diploid and/or polyploid cells.

9. A method according to any preceding claim, **characterized in that** the tumor cells are microsatellite stable or microsatellite instable.

10. An in vitro use of *NAV3* gene or a fragment thereof for predicting tumor initiation, tumor progression and/or carcinoma, genetic abnormalities of *NAV3* indicating tumors of epithelial origin.

11. An in vitro use of genetic markers at 12q21.2 for predicting tumor initiation, tumor progression and/or carcinoma, **characterized by** detecting the presence or absence of genetic abnormalities of *NAV3,* said genetic abnormalities indicating tumors of epithelial origin.

12. A use according to claim 11, **characterized in that** the genetic markers are D12S326 and/or rs1852464.

## Patentansprüche

1. *In-vitro*-Verfahren zum Vorhersagen von Tumorinitiierung, Tumorprogression und/oder eines Karzinoms, **gekennzeichnet durch** Nachweisen der Anwesenheit oder der Abwesenheit von genetischen Anomalien an 12q21.2 in einem Neuron-Navigator-3 (*NAV3*) Gen oder einem Fragment davon, wobei die Anwesenheit der genetischen Anomalien eine Initiierung oder Progression von Tumoren epithelialen Ursprungs und/oder eines Karzinoms in einer biologischen Probe anzeigt.

2. *In-vitro*-Verfahren zum Identifizieren einer Person mit Potenzial zum Entwickeln eines Karzinoms epithelialen Ursprungs, wobei das Verfahren den Nachweis von genetischen Anomalien an 12q21.2 in einem Neuron-Navigator-3 (*NAV3*) Gen oder einem Fragment davon umfasst, wobei die genetischen Anomalien Tumore epithelialen Ursprungs anzeigen.

3. ***In-vitro***-Verfahren zum Vorhersagen der Progression von Karzinomen epithelialen Ursprungs und/oder der Transformation davon in eine aggressive Variante, **gekennzeichnet durch** Nachweisen von genetischen Anomalien an 12q21.2 in einem Neuron-Navigator-3 *(NAV3)* Gen oder einem Fragment davon, wobei Anomalien die Wahrscheinlichkeit anzeigen, ein Karzinom zu entwickeln.

4. Verfahren gemäß einem vorhergehenden Anspruch, **dadurch gekennzeichnet, dass** es sich bei dem Tumor epithelialen Ursprungs um ein Adenom und/oder ein Karzinom handelt.

5. Verfahren gemäß einem vorhergehenden Anspruch, **dadurch gekennzeichnet, dass** der Tumor epithelialen Ursprungs sich im Kolon, Rektum, in der Lunge, Harnblase, Brust oder in Plattenepithel- oder Basalzellen befindet.

6. Verfahren gemäß einem vorhergehenden Anspruch, **dadurch gekennzeichnet, dass** genetische Anomalien durch Fluoreszenz-in-situ-Hybridisierung (FISH) bestimmt werden.

7. Verfahren gemäß den Ansprüchen 1-6, **gekennzeichnet durch** Bestimmen des Verlusts der Heterozygotie ("loss of heterozygosity", LOH) des *NAV3* Gens oder eines funktionellen Fragments davon, wobei LOH von *NAV3* auf Tumorprogression hinweist.

8. Verfahren gemäß den Ansprüchen 1 - 7, **dadurch gekennzeichnet, dass** genetische Anomalien des *NAV3* Gens in haploiden, diploiden und/oder polyploiden Zellen bestimmt werden.

9. Verfahren gemäß einem vorhergehenden Anspruch, **dadurch gekennzeichnet, dass** die Tumorzellen mikrosatellitenstabil oder mikrosatelliteninstabil sind.

10. *In*-*vitro-*Verwendung des *NAV3* Gens oder eines Fragments davon zum Vorhersagen von Tumorinitiierung, Tumorprogression und/oder eines Karzinoms, wobei genetische Anomalien von *NAV3* Tumore epithelialen Ursprungs anzeigen.

11. *In-vitro*-Verwendung von genetischen Markern an 12q21.2 zum Vorhersagen von Tumorinitiierung, Tumorprogression und/oder eines Karzinoms, **gekennzeichnet durch** Nachweisen der Anwesenheit oder Abwesenheit von genetischen Anomalien von *NAV3,* wobei die genetischen Anomalien Tumore epithelialen Ursprungs anzeigen.

12. Verwendung gemäß Anspruch 11, **dadurch gekennzeichnet, dass** es sich bei den genetischen Markern um D12S326 und/oder rs1852464 handelt.

## Revendications

1. Procédé in vitro permettant de prévoir le début d'une tumeur, la progression d'une tumeur et/ou un carcinome, **caractérisé par** la détection de la présence ou de l'absence d'anomalies génétiques dans la région 12q21.2 d'un gène *NAV3 (neuron navigator* 3) ou d'un de ses fragments, la présence desdites anomalies génétiques étant le signe du début ou de la progression de tumeurs d'origine épithéliale et/ou d'un carcinome dans un échantillon biologique.

2. Procédé in vitro permettant d'identifier un individu susceptible de développer un carcinome d'origine épithéliale, ledit procédé comprenant la détection d'anomalies génétiques dans la région 12q21.2 d'un gène *NAV3 (neuron navigator 3)* ou d'un de ses fragments, lesdites anomalies génétiques étant le signe de tumeurs d'origine épithéliale.

3. Procédé in vitro permettant de prévoir la progression de carcinomes d'origine épithéliale et/ou leur transformation en variante agressive, **caractérisé par** la détection d'anomalies génétiques dans la région 12q21.2 d'un gène *NAV3 (neuron navigator 3)* ou d'un de ses fragments, lesdites anomalies indiquant la probabilité de développer un carcinome.

4. Procédé selon l'une quelconque des revendications précédentes, **caractérisé en ce que** la tumeur d'origine épithéliale est un adénome et/ou un carcinome.

5. Procédé selon l'une quelconque des revendications précédentes, **caractérisé en ce que** la tumeur d'origine épithéliale se trouve dans le côlon, le rectum, le poumon, la vessie, le sein ou dans des cellules squameuses ou basales.

6. Procédé selon l'une quelconque des revendications précédentes, **caractérisé en ce que** les anomalies génétiques sont déterminées par la technique d'hybridation *in situ* en fluorescence (FISH).

7. Procédé selon les revendications 1 à 6, **caractérisé par** la détermination de la perte d'hétérozygotie (LOH) du gène *NAV3* ou d'un de ses fragments fonctionnels, la LOH de *NAV3* étant le signe de la progression d'une tumeur.

8. Procédé selon les revendications 1 à 7, **caractérisé en ce que** les anomalies génétiques du gène *NAV3* sont déterminées dans des cellules haploïdes, diploïdes et/ou polyploïdes.

9. Procédé selon l'une quelconque des revendications précédentes, **caractérisé en ce que** les cellules tumorales sont à microsatellites stables ou à microsatellites instables.

10. Utilisation in vitro du gène *NAV3* ou d'un de ses fragments pour prévoir le début d'une tumeur, la progression d'une tumeur et/ou un carcinome, des anomalies génétiques de *NAV3* étant le signe de tumeurs d'origine épithéliale.

11. Utilisation in vitro de marqueurs génétiques dans la région 12q21.2 pour prévoir le début d'une tumeur, la progression d'une tumeur et/ou un carcinome, **caractérisée par** la détection de la présence ou de l'absence d'anomalies génétiques de *NAV3,* lesdites anomalies génétiques étant le signe de tumeurs d'origine épithéliale.

12. Utilisation selon la revendication 11, **caractérisée en ce que** les marqueurs génétiques sont D12S326 et/ou rs1852464.
